# EUROPEAN PATENT APPLICATION

(11) **EP 0 606 497 A1**
(43) Date of publication of application: **20.07.1994**
(21) Application number: 93916234.3
(22) Date of filing: 27.07.1993
(51) Int. Cl.: A61K 35/78

(54) **REMEDY FOR BIOTOXIN TYPE BACTERIAL INTESTINAL INFECTIOUS DISEASES**

(30) Priority: 31.07.1992 JP 223653/92
(71) Applicant: TSUMURA & CO., Chuo-ku, Tokyo 103 (JP)
(72) Inventor: NODA, Masatoshi, Yotsukaido-shi, Chiba-ken 284 (JP); NISHIYA, Hajime, Edogawa-ku, Tokyio 133 (JP); YAMAGUCHI, Morimichi, Toshima-ku, Tokyo 170 (JP)
(74) Representative: Böhm, Brigitte, Dipl.-Chem. Dr.
(86) International application number: JP9301058
(87) International publication number: WO9403193

(57) **Abstract**

A remedy for biotoxin type bacterial intestinal infectious diseases containing an active ingredient comprising at least one crude drug selected from the group consisting of rhubarb, cinnamon bark, coptis rhizome and ephedra herb, extracts of these drugs, Chinese medicines containing them as the ingredient, or extracts of these medicines. The remedy has a cholera toxin resistance and is useful for treating biotoxin type bacterial intestinal infectious diseases such as cholera and traveler's diarrhea.

## Description

### Field of the Invention

The present invention relates to a pharmaceutical preparation which is effective for the treatment of toxin (in vivo ) mediated type infectious diseases in the intestine caused by bacteria.

### Background of the Invention

Intestinal infectious diseases are caused by organisms such as protists, eueumycetes, viruses, microorganisms, etc., and in particular, the microorganisms are major agents. According to the onset mechanism, the microorganisms causing the intestinal infectious diseases are roughly divided into 3 types, ① tissue-invading type, ② toxin (in vivo) mediated type (infectious toxin type), and ③ toxin (in vitro) mediated type. Among them, the toxin (in vivo) mediated type infectious diseases in the intestine by bacteria are caused by microorganisms which orally entered the intestine where they multiply and produce toxins, as is the case with diarrhea by Vibrio cholerae and enterotoxigenic Escherichia coli, and typical diseases are cholera and traveler's diarrhea (The Journal of Practical Pharmacy, 42, 1105 (1991)).

Cholera is a disease accompanied by the principal symptom of severe diarrhea, caused by infection of Vibrio cholerae, and this disease is prevalent all over the world, particularly Asia and Africa. The disease is highly fatal and very acute with a short course from the onset of diarrhea, via the progress of disease, to the death. At present, transfusion as symptomatic treatment is all that is available for the treatment of cholera, and no fundamental method has been developed for such treatment.

In recent years, it has been noteworthy that the cholera toxin produced by Vibrio cholerae is made nontoxic by inhibition and suppression (anti-cholera toxin activity) of the activity of the toxin. However, no pharmaceutical preparation with anti-cholera toxin activity has been discovered yet, and developments in a substance with such activity have been desired.

Enterotoxigenic Escherichia coli is a bacterium producing heat-labile enterotoxin or heat-stable enterotoxin, or both. This bacterium, found in 1971 by Sack et al., is a principal agent of tourist's diarrhea and is one of the most essential bacteria responsible for diarrhea (The Journal of Practical Pharmacy, 42, 1105 (1991)). Said heat-stable enterotoxin is considerably similar to the cholera toxin in the molecular structure, antigenecity and biological activities (The Bimonthly Journal of Microorganism, 3, 248 (1987)). Hence, it is believed that a pharmaceutical preparation with the anti-cholera toxin activity also makes the heat-labile enterotoxin nontoxic by inhibiting and suppressing its activity.

### Detailed Description of the Invention

To solve the above problem, the present inventors made extensive researches on a wide variety of crude drugs and herb medicines, and as a result, they found that at least one crude drug selected from the group consisting of rhubarb rhizome, cinnamon bark, coptis rhizome and ephedra herb or an extract thereof, as well as a herb medicine containing the same as a constituent ingredient or an extract thereof, possess the anti-cholera activity.

That is, the present invention encompasses:
(1) An agent for the treatment of toxin (in vivo) mediated type infectious diseases in the intestine caused by bacteria, comprising as an active ingredient at least one crude drug (hereinafter referred to as "the crude drug of the present invention") selected from the group consisting of rhubarb rhizome, cinnamon bark, coptis rhizome and ephedra herb or an extract thereof.
(2) An agent according to item (1), wherein the active ingredient is a herb medicine (hereinafter referred to as "the herb medicine of the present invention") containing as a constituent prescription at least one crude drug selected from the group consisting of rhubarb rhizome, cinnamon bark, coptis rhizome and ephedra herb or an extract thereof or is an extract thereof.
(3) An agent according to item (1), wherein the active ingredient is rhubarb rhizome or an extract thereof.
(4) An agent according to item (1), wherein the active ingredient is cinnamon bark or an extract thereof.
(5) An agent according to item (1), wherein the active ingredient is coptis rhizome or an extract thereof.
(6) An agent according to item (1), wherein the active ingredient is ephedra herb or an extract thereof.
(7) An agent according to item (2), wherein the active ingredient is daio-kanzo-to or an extract thereof.
(8) An agent according to item (2), wherein the active ingredient is keishi-ka-shakuyaku-daio-to or an extract thereof.
(9) An agent according to item (2), wherein the active ingredient is mao-bushi-saishin-to or an extract thereof.
(10) An agent according to item (2), wherein the active ingredient is oren-gedoku-to or an extract thereof.
(11) An agent according to one of items (1)-(10), which is an agent for the treatment of cholera.

The crude drugs of the present invention, rhubarb rhizome, cinnamon bark, coptis rhizome and ephedra herb, are known to have such actions as sedation, antispasm, anti-inflammation, etc., but are not known to possess the anti-cholera toxin activity.

As the herb medicines of the present invention, mention is made of kakkon-to, kakkon-to-ka-senkyu-shin'i, otsuji-to, anchu-san, hachimi-jio-gan, dai-saiko-to, saiko-keishi-to, saiko-keishi-kankyo-to, saiko-ka-ryukotsu-borei-to, hange-shashin-to, oren-gedoku-to, goshaku-san, keishi-ka-jutsu-bu-to, sho-seiryu-to, keishi-bukuryo-gan, keishi-ka-ryukotsu-borei-to, mao-to, daio-botampi-to, moku-boi-to, toki-shigyaku-ka-goshuyu-shokyo-to_{,} ryo-kei-jutsu-kan-to, keishi-to, juzen-taiho-to, keigai-rengyo-to, juncho-to, yokuinin-to, unsei-in, seijo-bofu-to, keishi-ka-shakuyaku-to, tokaku-joki-to, bofu-tsusho-san, goshaku-san, sha-kanzo-to, nyoshin-san, saikan-to, choi-joki-to, saiko-seikan-to, keishi-ninjin-to, daio-kanzo-to, ji-daboku-ippo, chikujo-untan-to, sho-kenchu-to, toki-to, tsu-do-san, unkei-to, gosha-jinki-gan, ninjin-yoei-to, san'o-shashin-to, sairei-to, irei-to, inchin-gorei-san, oren-to, toki-kenchu-to, keishi-ka-shakuyaku-daio-to, mao-bushi-saishin-to, inchinko-to, etc., among which particularly preferable are daio-kanzo-to, keishi-ka-shakuyaku-daio-to_{,} mao-bushi-saishin-to and oren-gedoku-to.

The formulation of the herb medicines of the present invention is described in classics such as "Gaidai-hi-yoho," "Shokan-ron," "Kinki-yoryaku," etc.. For example, in daio-kanzo-to, keishi-ka-shakuyaku-daio-to_{,} mao-bushi-saishin-to and oren-gedoku-to, crude drugs are formulated generally in the following ranges although there are some variations. The herb medicines of the present invention are known to be applied to a wide variety of diseases such as constipation, acute enteritis, hypertension, etc., but are not known to be useful as agents for the treatment of in vivo toxin type infectious diseases in the intestine caused by bacteria, such as cholera, tourist's diarrhea, etc.

### Daio-kanzo-to

4.0 parts by weight of rhubarb rhizome
1.0 to 2.0 parts by weight of glycyrrhiza root

### Keishi-ka-shakuyaku-daio-to

6.0 parts by weight of peony root
4.0 parts by weight of cinnamon bark
4.0 parts by weight of jujube fruit
2.0 parts
by weight of glycyrrhiza root
1.0 to 2.0 parts by weight of rhubarb rhizome
1.0 to 4.0 parts by weight of dried ginger rhizome

### Mao-bushi-saishin-to

4.0 parts by weight of ephedra herb
3.0 parts by weight of asiasarum root
0.5 to 1.0 part by weight of heat processed aconite tuber powder

### Oren-gedoku-to

3.0 parts by weight of scutellaria root
1.5 to 2.0 parts by weight of coptis rhizome
2.0 to 3.0 parts by weight of gardenia fruit
1.5 to 3.0 parts by weight of phellodendron bark
As extracts of the crude drugs or herb medicines of the present invention, mention is made of those extracted with a wide variety of aqueous solvents, and those extracted with water are preferably used. Typical preparation of extracts involves extracting the above crude drugs or herb medicines with 10-fold excess hot water and then filtering the resultant extract. These extracts may dried if necessary for use as dry powder.

Specific examples for the preparation of extracts are illustrated below:

### Specific Example 1: Daio-kanzo-to

240 g of purified water was added to mixed crude drugs (daio-kanzo-to: 24.0 g) consisting of 16.0 g of rhubarb rhizome and 8.0 g of glycyrrhiza root, and the sample was extracted by heating at 100 °C for 1 hour. The resultant extract was filtered and then spray-dried, to yield 6.0 g dry extract powder.

### Specific Example 2: Keishi-ka-shakuyaku-daio-to

190 g of purified water was added to mixed crude drugs (keishi-ka-shakuyaku-daio-to: 19.0 g) consisting of 6.0 g of peony root, 4.0 g of cinnamon bark, 4.0 g of jujube fruit, 2.0 g of glycyrrhiza root, 2.0 g of rhubarb rhizome and 1.0 g of dried ginger rhizome, and the sample was extracted by heating at 100 °C for 1 hour. The resultant extract was filtered and then spray-dried, to yield 4.0 g dry extract powder.

### Specific Example 3: Mao-bushi-saishin-to

240 g of purified water was added to mixed crude drugs (mao-bushi-saishin-to: 24.0 g) consisting of 12.0 g of ephedra herb, 9.0 g of asiasarum root and 3.0 g of heat processed aconite tuber powder, and the sample was extracted by heating at 100 °C for 1 hour. The resultant extract was filtered and then spray-dried, to yield 4.5 g dry extract powder.

### Specific Example 4: Oren-gedoku-to

255 g of purified water was added to mixed crude drugs (oren-gedoku-to: 25.5 g) consisting of 9.0 g of scutellaria root, 6.0 g of coptis rhizome, 6.0 g of gardenia fruit and 4.5 g of phellodendron bark, and the sample was extracted by heating at 100 °C for 1 hour. The resultant extract was filtered and then spray-dried, to yield 4.5 g dry extract powder.

### Specific Example 5: Rhubarb rhizome

240 g of purified water was added to 24 g of rhubarb rhizome, and the sample was extracted by heating at 100 °C for 1 hour. The resultant extract was filtered and then spray-dried, to yield 8.6 g dry extract powder.

### Specific Example 6: Cinnamon bark

240 g of purified water was added to 24 g of cinnamon bark, and the sample was extracted by heating at 100 °C for 1 hour. The resultant extract was filtered and then spray-dried, to yield 2.6 g dry extract powder.

### Specific Example 7: Coptis rhizome

240 g of purified water was added to 24 g of coptis rhizome, and the sample was extracted by heating at 100 °C for 1 hour. The resultant extract was filtered and then spray-dried, to yield 5.0 g dry extract powder.

### Specific Example 8: Ephedra herb

240 g of purified water was added to 24 g of ephedra herb, and the sample was extracted by heating at 100 °C for 1 hour. The resultant extract was filtered and then spray-dried, to yield 4.8 g dry extract powder.

### Specific Example 9: Daio-kanzo-to

24 liters of purified water was added to mixed crude drugs (daio-kanzo-to: 2,400 g) consisting of 1,600 g of rhubarb rhizome and 800 g of glycyrrhiza root, and the sample was extracted for 1 hour by heating at 100 °C. For removal of residues, the resultant extract was centrifuged to give 20 liters of solution.

This solution was filtered through a 0.3 µm membrane filter (manufactured by Toyo Roshi Co., Ltd.) while the microorganisms present were removed by filtration. The resultant filtrate was subjected to ultrafiltration through Diafilter G-10T (manufactured by Bioengineering Co.: fractionation molecular weight of 10,000). For ultrafiltration, a membrane of 152 mm in diameter was attached to the bottom of a vessel with a capacity of 2.0 liters, into which the sample was introduced. As the sample was concentrated under a pressure of 3 kg/cm², about 2 liters of purified water was repeatedly introduced. As a result, 20 liters of filtrate was obtained in the ultrafiltration.

The anti-cholera toxin activity possessed by the active ingredients of the present invention is described with reference to the following examples.

### Experimental Example 1

### CHO (Chinese hamster ovary) cell assay

In this experiment, the crude drug or herb medicine of the present invention was added in an amount of 10 mg/ml and then extracted at 37 °C for 30 min. The extract thus obtained was centrifuged to give a supernatant which was then diluted to a predetermined concentration.

10 ng/ml cholera toxin and each crude drug or herb medicine (extract supernatant) were added to CHO cells (2.5×10³ cells/ml) and allowed to stand at 37 °C for 17 hours. The inhibitory effect on the change by cholera toxin in cells from egg shapes to spindle shapes was evaluated for the determination of percent inhibition.

### Experimental Example 2

### Agmatine assay

1 µg of A-subunit of cholera toxin, 0.7 mg of agmatine, 0.0375 µCi [¹⁴C] nicotinamide adenine dinucleotide, and each crude drug or herb medicine (extract supernatant) obtained in Experimental Example 1 were mixed, and the mixture was kept at 30 °C for 3 hours, and the formed product [¹⁴C] ADP (adenosine diphosphate)-ribosylated agmatine was measured. The percent inhibition of the ADP-ribosyltransferase activity of cholera toxin by the active ingredient(s) of the present invention was determined based on the formation of [¹⁴C] ADP-ribosylated agmatine.

The results obtained in Experimental Examples 1 and 2 are set forth in Table 1 where the concentration is the final concentration of each crude drug or herb medicine in a solution from which the corresponding extract supernatant was obtained.

**Table 1**

| crude drug/herb medicine | concentration ( µg/ml) | CHO cell assay (inhibition %) | agmatine assay (inhibition %) |
|---|---|---|---|
| daio-kanzo-to | 3 | 98 | 100 |
| keishi-ka-shakuyaku-daio-to | 9 | 98 | 98 |
| mao-bushi-saishin-to | 3 | 98 | 98 |
| | 200 | | 99 |
| | 667 | | 98 |
| oren-gedoku-to | 3 | 98 | 6 |
| rhubarb rhizome | 1 | 99 | 98 |
| | 67 | | 99 |
| cinnamon bark | 1 | 99 | 99 |
| coptis rhizome | 3 | 98 | 32 |
| ephedra herb | 1 | 50 | |
| | 2 | 99 | |
| | 6.7 | | 53 |
| | 20 | | 97 |
| | 67 | | 98 |
| | 200 | | 98 |
| | 667 | | 99 |

As can be seen from the above results, the active ingredients of the present invention act directly on the cholera toxin, and significantly inhibit ADP-ribosyltransferase activity, indicating that they possess anti-cholera toxin activity. Hence, the agents of the present invention are effective for the treatment of toxin (in vivo ) mediated type infectious diseases in the intestine caused by bacteria, such as cholera, tourist's diarrhea, etc..

The active ingredients of the present invention can be used with high reliability because of their long history and confirmed safety. For example, mice and rats orally given the present ingredients survived at a critical dosage of 15 g/kg, which indicates their considerably high safety.

The pharmaceutical manufacturing and dosage of the present agents are described below.

As an agent for the treatment of infectious diseases, the crude drug or herb medicine of the present invention can be formed as such or in the form of an extract thereof into a pharmaceutical preparation in combination with a conventional pharmaceutical carrier. The form of the agent administered can be suitably selected as necessary, including but not limited to tables, capsules, granules, fine granules, powder, etc., for oral administration or injection liquid, suppositories, etc., for parenteral administration.

To demonstrate the desired effect, the present agent is administered into an adult preferably at a dosage of 3-15 g active ingredient daily at suitable intervals, depending on the age, weight and disease severeness of the patient.

A preparation for oral administration is manufactured in a usual manner using starch, milk sugar, white sugar, mannitol, carboxymethylcellulose, corn starch, inorganic salts, etc.

This type of pharmaceutical preparation can use binding agents, disintegrators, surface active agents, lubricants, fluidity promoters, correctives, coloring agents, spices, etc., in addition to the vehicles enumerated above. As specific examples, binding agents are starch, dextrin, powder of gum arabic, gelatin, hydroxypropyl starch, methyl cellulose, carboxymethylcellulose sodium, hydroxypropylcellulose, crystalline cellulose, ethyl cellulose, polyvinylpyrrolidone, and macrogol; disintegrators are starch, hydroxypropyl starch, carboxymethylcellulose sodium, carboxycellulose calcium, carboxymethyl cellulose, low-substituted hydroxypropyl cellulose; surface active agents are sodium lauryl sulfate, soybean lecithin, sucrose fatty acid ester, and Polysolvate 80; lubricants are talc, waxes, hydrogenated vegetable oils, sucrose fatty acid esters, magnesium stearate, calcium stearate, aluminum stearate, polyethylene glycol; and fluidity promoters are light silicic anhydride, dried gel of aluminum hydroxide, synthetic aluminum silicate, and magnesium silicate.

The agents of the invention can be administered in the form of suspension, emulsion, syrup and elixir, each form of which may contain correctives and coloring agents.

A preparation for parenteral administration is manufactured in a usual manner, which may make use of diluents such as distilled water for injection, physiological saline, aqueous glucose solution, vegetable oil for injection, sesame oil, peanut oil, soybean oil, corn oil, polypropylene glycol, polyethylene glycol, etc. Additives such as disinfectants, antiseptics and stabilisers may be incorporated as necessary. For keeping stability, such a parenteral preparation can be lyophilized in vials etc. by conventional techniques to reconstitute a liquid preparation just before use. Furthermore, additives such as isotonic agents, stabilizers, disinfectants and analgesia agents may also be incorporated as necessary.

The present agents for the treatment of toxin (in vivo) mediated type infectious diseases in the intestine caused by bacteria possess the anti-cholera toxin activity, and are useful for treatment of diseases such as cholera, tourist's diarrhea, etc.

### Examples

Hereinafter, the present invention is described in more detail with reference to Examples, which however are not intended to limit the scope of the invention.

### Example 1

| | |
|---|---|
| ① corn starch | 21 g |
| ② crystalline cellulose | 10 g |
| ③ carboxymethylcellulose calcium | 7 g |
| ④ light silicic anhydride | 1 g |
| ⑤ magnesium stearate | 1 g |
| ⑥ daio-kanzo-to obtained in Specific Example 1 | 160 g |
| | Total 200 g |

According to the above formulation, ingredients ① to ⑥ were uniformly mixed and compression-molded into tablets (200 mg per tablet) using a tableting machine.

One tablet contains 160 mg of daio-kanzo-to obtained in Specific Example 1. 20-80 tablets are orally administered daily into an adult at suitable intervals.

### Example 2

| | |
|---|---|
| ① corn starch | 29 g |
| ② magnesium stearate | 2 g |
| ③ carboxymethylcellulose calcium | 8 g |
| ④ light silicic anhydride | 1 g |
| ⑤ keishi-ka-shakuyaku-daio-to obtained in Specific Example 2 | 160 g |
| | Total 200 g |

According to the above formulation, ingredients ① to ⑤ were uniformly mixed, then compression-molded, ground with a crusher and screened, whereby granules were obtained.

1 g of the granules contains 800 mg of keishi-ka-shakuyaku-daio-to obtained in Specific Example 2, and 4-18 g of the granules is orally administered daily into an adult at suitable intervals.

### Example 3

| | |
|---|---|
| ① corn starch | 19 g |
| ② light silicic anhydride | 1 g |
| ③ mao-bushi-saishin-to obtained in Specific Example 3 | 180 g |
| | Total 200 g |

According to the above formulation, ingredients ① to ③ were mixed uniformly, and 200 mg of the mixture was introduced into No. 2 capsule.

One capsule contains 180 mg of mao-bushi-saishin-to obtained in Specific Example 3, and 20-80 capsules are orally administered into an adult at suitable intervals.

### Example 4

300 g of (pyrogen-free) alanine was dissolved in 20 liters of daio-kanzo-to obtained in Specific Example 9. This solution was then pipetted into 900 vials and lyophilized, so that powder injection agent was obtained. 1 vial contains 406 mg of the lyophilized sample which forms a transparent solution when dissolved in 10 ml of purified water. This aqueous solution passed the pyrogen test under the Japanese Pharmacopoeia.

## Claims

1. An agent for the treatment of toxin (in vivo) mediated type infectious diseases in the intestine caused by bacteria, comprising as an active ingredient at least one crude drug selected from the group consisting of rhubarb rhizome, cinnamon bark, coptis rhizome and ephedra herb or an extract thereof.

2. An agent according to claim 1, wherein the active ingredient is a herb medicine containing as a constituent prescription at least one crude drug selected from the group consisting of rhubarb rhizome, cinnamon bark, coptis rhizome and ephedra herb or an extract thereof or is an extract thereof.

3. An agent according to claim 1, wherein the active ingredient is rhubarb rhizome or an extract thereof.

4. An agent according to claim 1, wherein the active ingredient is cinnamon bark or an extract thereof.

5. An agent according to claim 1, wherein the active ingredient is coptis rhizome or an extract thereof.

6. An agent according to claim 1, wherein the active ingredient is ephedra herb or an extract thereof.

7. An agent according to claim 2, wherein the active ingredient is daio-kanzo-to or an extract thereof.

8. An agent according to claim 2, wherein the active ingredient is keishi-ka-shakuyaku-daio-to or an extract thereof.

9. An agent according to claim 2, wherein the active ingredient is mao-bushi-saishin-to or an extract thereof.

10. An agent according to claim 2, wherein the active ingredient is oren-gedoku-to or an extract thereof.

11. An agent according to one of claims 1-10, which is an agent for the treatment of cholera.
